# EUROPEAN PATENT APPLICATION

(11) **EP 4 285 869 A1**
(43) Date of publication of application: **06.12.2023**
(21) Application number: 22177063.9
(22) Date of filing: 02.06.2022
(51) Int. Cl.: A61F 2/38

(54) **TIBIAL TRAY, TIBIAL INSERT AND AN ARTIFICIAL KNEE JOINT**

(71) Applicant: CeramTec GmbH, 73207 Plochingen (DE)
(72) Inventor: BISSCHOP, Bastian, 73033 Göppingen (DE); GEBERT DE UHLENBROCK, Anne, 91367 Weißenohe (DE); HÄUßLER, Kim Lars, 70176 Stuttgart (DE); MAIER, Dennis, 73312 Geislingen (DE); UPMANN, Carsten, 73730 Esslingen (DE)
(74) Representative: Maikowski & Ninnemann Patentanwälte Partnerschaft mbB

(57) **Abstract**

The invention relates to a tibial tray (20) with a proximal surface (P), the proximal surface (P) comprising at least two, in particular exactly two central fixation elements (1) for enabling a connection with a tibial insert (30) as a further part of an artificial knee joint, wherein the at least two central fixation elements (1) are protrusions from the proximal surface (P) in the proximal direction and the at least two central fixation elements (1) are positioned symmetrically and / or parallel to a medial plane (M) of the proximal surface (P), the medial plane (M) being perpendicular to the proximal surface (P) and intersecting the proximal surface (P) in the middle between two most lateral points (L1, L2) of the proximal surface (P). The invention also relates to a tibial insert (30) and an artificial knee joint.

## Description

The invention relates to a tibial tray with the features of claim 1 and a tibial insert with the features of claim 13 and an artificial knee joint with the features of claim 16.

Joint replacements, in particular artificial knee joint replacements have been known for quite some time. In the case of an artificial knee joint it is known that a tibial tray part is connected with the proximal end of the tibia of the patient. A femur part is connected with the distal end of the femur of the patient. A tibial insert is connected with the proximal surface of the tibial tray so that after the completion of the surgical procedure, the tibial insert is positioned between the distal surface of the femur part and the proximal surface of the distal tray.

A tibial tray and a tibial insert of this kind are e.g. described in EP 3 626 209 B1.

It is a challenge to provide a combination of a tibial tray and matching tibial insert so that the locally occurring mechanical loads are not exceeding certain design limits. This, in particular, is important when a ceramic material is used for the tibial tray.

The tibial tray with the features of claim 1 addresses these issues.

The tibial tray comprises a proximal surface, the proximal surface comprising at least two, in particular exactly two central fixation elements for enabling a connection with a tibial insert as a further part of artificial knee joint. The central fixation elements are protrusions from the proximal surface which can connect as form fitting and / or force locking with the tibial insert.

The at least two central fixation elements are designed as protrusions from the proximal surface in the proximal direction, i.e. the central fixation elements generally point away from the proximal surface in the direction of the tibial insert.

The at least two central fixation elements are positioned symmetrically and / or parallel to a medial plane of the proximal surface, the medial plane being perpendicular to the proximal surface and intersecting the proximal surface in the middle between two most lateral points of the proximal surface. The medial plane is dividing the proximal surface into two lateral halves. The at least two central fixation elements are oriented symmetrically and / or parallel to the medial plane.

The at least two central fixation elements configured as cylindrical protrusions and / or linear protrusions. Cylindrical means that the protrusion has e.g. a circular, elliptical cross-section. Linear means that the protrusion extends linearly on the proximal surface. At least one of the central fixation element can e.g. have a linear or cylindrical.

In one embodiment, the least two central fixation elements are symmetrically positioned relative to a frontal plane perpendicular to the medial plane. The frontal plane is dividing the proximal surface into an anterior and a posterior part. In particular, it is possible that the frontal plane is going through the most lateral points of the proximal surface.

Apart from the central fixation elements, the proximal surface can comprise further structures.

An anterior fixation element, in particular exactly one anterior fixation element, can be positioned at the anterior side of the proximal surface, the posterior side of the at least one anterior fixation element comprising a wall which in a plane parallel to the proximal surface is perpendicular to the medial plane of the proximal surface. This posterior wall can provide some connection elements for the tibial insert at the anterior side.

In one embodiment, the at least one anterior fixation element comprises at least one form fitting element, in particular, at least one cavity in the posterior wall of the at least one anterior fixation element. This is an example of a connection element for the tibial insert. Alternatively or in addition, the at least one channel goes through the at least one anterior fixation element for a match with a corresponding element from fitting element of the tibial insert.

In a further embodiment, the at least one form fitting element of the at least one anterior fixation element is positioned in a region in which the load is at less than 70%, in particular less than 50% from the maximum load. The loads (pressure, torque etc.) can be computed under various conditions. Generally, certain regions in the structures will show higher loads than others. One region will show the maximum load which is clearly defined. Regions which experience a load of less than 50% of that maximum value are potential locations for the at least one form fitting element.

There can also be a structure at the posterior end of the proximal plane. At least one, in particular exactly one, posterior fixation element can be positioned symmetrically to the medial plane of the proximal surface. The posterior fixation element comprises a posterior wall flush with the posterior rim of the tibial tray and / or comprises an anterior wall which is parallel to the posterior wall of the at least one anterior fixation element. The at least one posterior fixation element can comprise two sidewalls, an angle between the sidewalls and the anterior wall in a plane parallel to the proximal plane can be in the range between 5 and 85°, in particular in the range between 40 and 50°. Furthermore, the sidewalls of the at least one posterior element are tilted by an angle between 1 and 25°, in particular between 3 and 10° relative to a plane which is perpendicular to the proximal plane. This enables e.g. the formation of posterior block with inward tilted sidewalls and sidewalls converging to the anterior side. Such a posterior fixation element can be used as a fulcrum when attaching a tibial insert.

For a better hold of the tibial insert during assembly, the posterior wall of the at least one anterior fixation element and / or the anterior wall of the at least one posterior fixation element can comprise an undercut, in particular the undercut forming an angle between 10 and 30°, in particular 15°, with a plane perpendicular to the proximal surface.

In one embodiment, the height of the at least two central fixation elements, the at least one anterior fixation element and / or the at least one posterior fixation element extending from the proximal surface is in the range between 1 mm and 6 mm, in particular between 3 and 4,5 mm and / or wherein the anterior and / or posterior ends of the at least two central fixation elements are rounded. In particular, the height of the at least two central fixation elements, the height of the at least one anterior fixation element and / or the height of the at least one posterior fixation element above the proximal surface is constant or varies by maximally 10% from the minimal height. These features are instrumental for providing a secure connection with the tibial insert and / or allow an effective assembly.

The issues are also addressed by a tibial insert designed or configured to match the tibial tray as claimed.

To ensure a good connection with the tibial tray, the tibial insert can comprise one recess in the distal surface for the matching of at least one posterior fixation element, the recess comprising at least one stress relief notch at the junction of two walls of the recess. This prevents the build-up of mechanical stress sharp corners where two walls of the recess meet.

The secure connection can be enabled by at least one form fitting element of the tibial insert for engaging the at least one form fitting element of the at least one anterior fixation element of the tibial tray.

The issues are also addressed by an artificial knee joint comprising a claimed tibial tray according to and a claimed tibial insert, wherein there is a light press fit connection between the tibial insert and the at least two central fixation elements, the at least one anterior fixation element and the at least one posterior fixation element, in particular having a press fit in the range of 0 to 250 µm.

Embodiments of the invention are shown in the figures,
- Fig. 1: showing a perspective view of an artificial knee joint;
- Fig. 2: showing a perspective view of an embodiment of a tibial tray;
- Fig. 2A: same view of Fig. 2 highlighting reference frames;
- Fig. 2B: showing a variation of the embodiment in Fig. 2;
- Fig. 3A: showing a view of the proximal surface of an embodiment of a tibial tray;
- Fig. 3B: showing a view of the distal surface of a tibial insert matching the tibial tray shown in Fig. 3A;
- Fig. 4: showing a view of a proximal surface of the embodiment shown in Fig. 3A indicating a press fit
- Fig. 5A: showing the fitting of a tibial insert to a tibial tray from a perspective view
- Fig. 5B: the fitting of the tibial insert to the tibial tray in Fig. 5A in a sectional view;
- Fig. 6A: a detailed frontal view of a first embodiment of the tibial insert;
- Fig. 6B: a detailed frontal view of a second embodiment of the tibial insert;
- Fig. 7: a perspective view of a variation of the embodiment shown in Fig. 2.

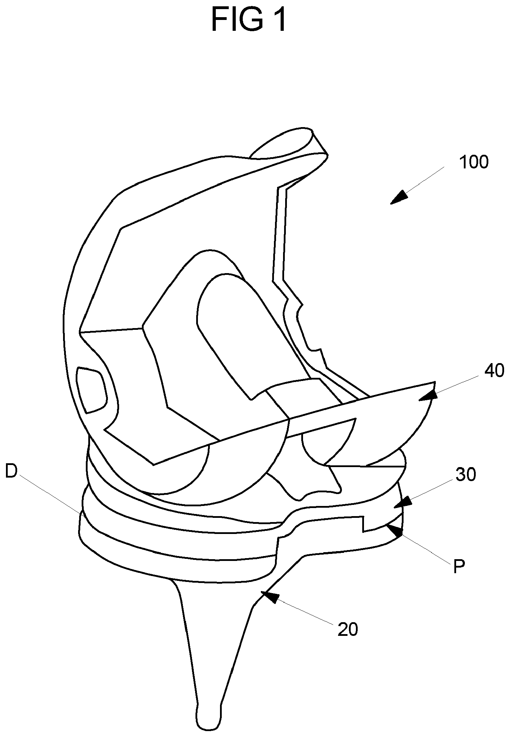

In Fig. 1 an artificial ceramic knee joint 100 is shown in a perspective view from a posterior position. In Fig. 1 a femur part 40, a tibial insert 30 and a tibial tray 20 of the metal-free artificial knee joint are shown. The complete artificial art knee joint 100 is metal-free, e.g. a combination of ceramic and polymeric materials. The tibial tray 20 can e.g. made from ceramic and the tibial insert 30 can be made from a polymeric material.

At the proximal position of the artificial knee joint a femur part 40 is shown with two condyles fitting into matching grooves in the proximal surface of a tibial insert 30 at the distal end of the femur part 40.

The tibial insert 30 is connected with a tibial tray 20, the distal surface D of the tibial insert 30 facing the proximal surface P of the tibial tray 20.

In the following, embodiments of the tibial tray 20 and the tibial insert 30 are described, in particular features of the proximal surface P of the tibial tray 20 and the matching distal surface D of the tibial insert 30.

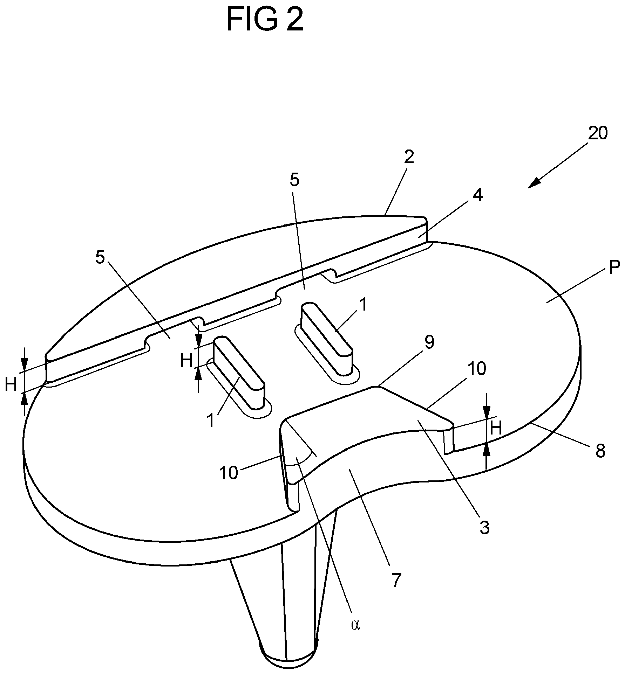

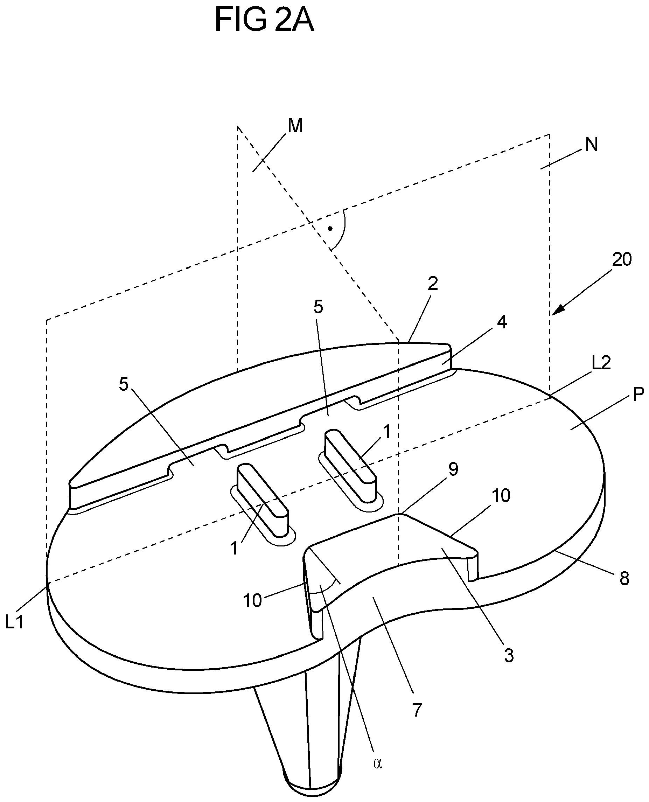

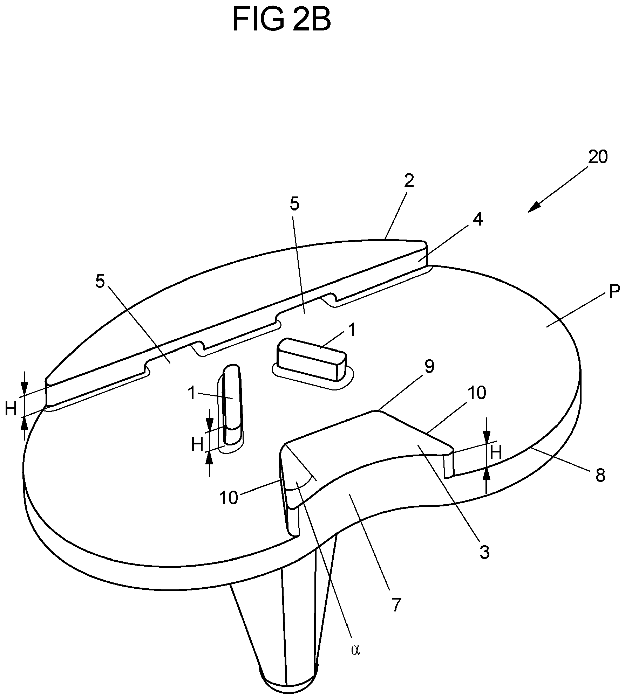

Fig. 2, 2A show a perspective view onto the proximal surface P of the tibial tray 20. In Fig. 2A the tibial tray is shown for reducing complexity without the reference numbers but with reference planes M, N which are used in the following to define the locations of features on the tibial tray 20.

Referring to Fig. 2A, a medial plane M is perpendicular to the planar proximal surface P and intersecting the proximal surface P in the middle between to most lateral points L1, L2 of the proximal surface P. A frontal plane N is perpendicular to the medial plane M.

In one embodiment of the tibial tray 20, the dimensions are grouped in different sizes. There are eight different sizes for the distance between the points L1 and L2. This is used to allow for artificial knee joints 100 for differently sized patients. The smallest distance between L1 and L2 is 60 mm. The other seven sizes have distances of 64 mm, 68 mm, 72 mm, 76 mm, 80 mm, 84 mm and 88 mm (i.e. using an increment of 4 mm). Other embodiments can use different absolute sizes and / or different increments, the increments do not have to be identical.

In the extent of the proximal surface P in the direction perpendicular to the distance between L1 and L2, the corresponding sizes are 38,7 mm, 41,3 mm, 43,9 mm, 46,5 mm, 49,0 mm, 51,6 mm, 54,2 mm and 56,8 mm. Other embodiments can use different absolute sizes and / or different increments, again, the increments do not have to be identical.

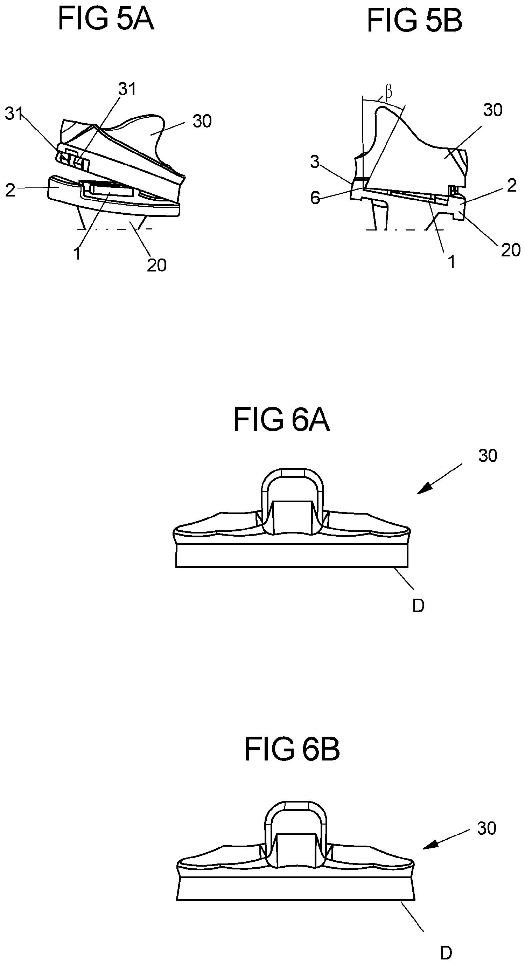

The proximal surface P of the tibial tray 20 is structured so that a corresponding tibial insert 30 (see e.g., Fig. 1, 6) can be securely fastened to the tibial tray 20 (see Fig. 5A, B). The planar proximal surface P itself is polished with a mean roughness of Ra=0,1 µm and an evenness of 0,1. In other embodiments the mean roughness is at less than 5 µm, in particular at less than 2 µm.

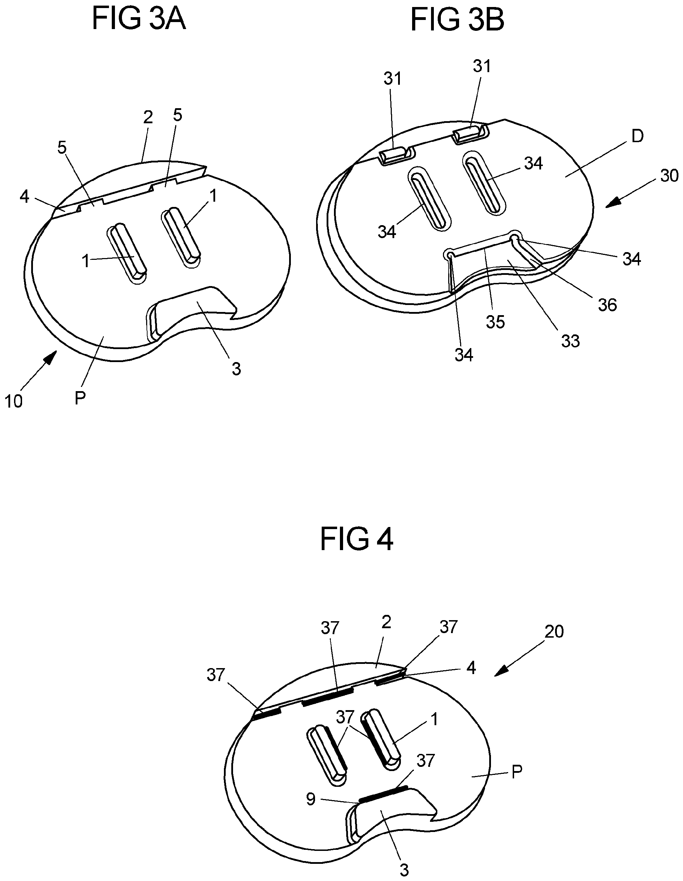

In the following, different aspects of the structures 1, 2, 3 on the planar proximal surface P are described. The structures provide a symmetric, flat plateau with island-like fixation elements 1, 2, 3 for the tibia insert 30 connection using a press fit (see Fig. 4).

In the embodiment shown in Fig. 2, the proximal surface P comprises two central fixation elements 1. As will be described below, those two central fixation elements enable a secure connection with the tibial insert (see Fig. 3B) as a further part of the artificial knee joint 100.

In the embodiment shown the two central fixation elements 1 are linear protrusions, protruding away from the proximal surface P in the proximal direction, so that they can match corresponding grooves 34 in the tibial insert 30 (see Fig. 3B). In other embodiments the central fixation elements 1 could have a cylindrical shape.

The two central fixation elements 1 are positioned symmetrically and parallel to the medial plane M.

In an embodiment shown in Fig. 2B, the two central fixation elements 1 are still symmetric to the medial plane M, but not quite parallel as they are angled by less than 5° relative to the median plane M, so that the two linear fixation elements 1 show some convergence towards the anterior side.

In other embodiments it is also possible that the two central fixation elements 1 are asymmetric to the median plane M but parallel to each other.

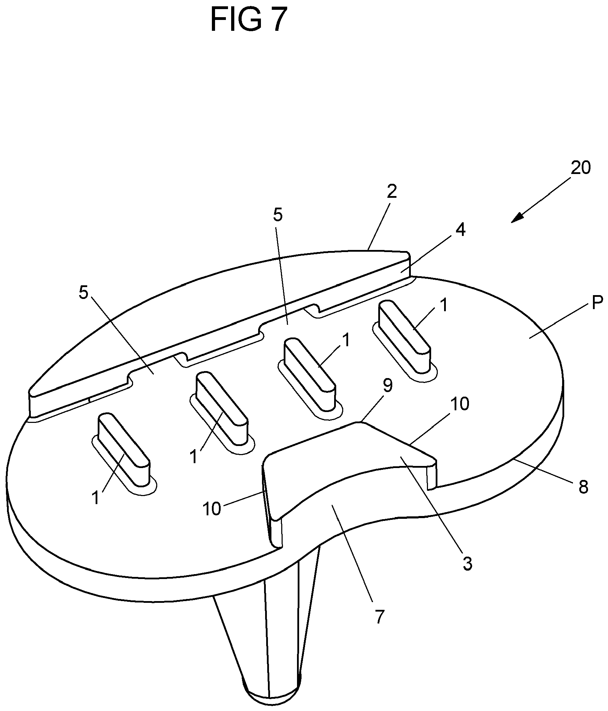

As it is shown in Fig. 7, in a different embodiment, more than two central fixation elements 1 can be used. Otherwise, the embodiment of Fig. 7 is comparable to the one described in connection with Fig. 2.

In the embodiment shown in Fig. 2, the two central fixation elements 1 are symmetrically positioned relative to the frontal plane N perpendicular to the medial plane M. In this embodiment, but not necessarily, the frontal plane N is going through the most lateral points L1, L2 of the proximal surface P.

The two central fixation elements 1 have the form of parallelepiped the anterior and the posterior ends of the central fixation elements 1 are rounded.

The two central fixation elements 1 have a height H extending in the embodiment shown 3,8 mm from the proximal surface P of the tibial tray 20. In other artificial knee joints 100, the height H can be up to 6 mm. In the embodiment shown, the height H of the central fixation elements 1 is constant. In other embodiments, the height H can vary relative to the proximal surface P. In other embodiments, the height H of the at least two central fixation elements 1 over the proximal surface varies by maximally 10% from the minimal height.

In the embodiment shown in Fig. 2, the distance between the two central fixation elements 1 is 10,5 mm as measured between the interior walls and 15,5 mm measured between the exterior walls. As the two central fixation elements 1 are parallel to each other, the width of each of the central fixation elements is 2,5 mm. The distance between the central fixation elements is chosen here to be as large as possible to provide a large torque and to prevent a shearing off of polyethylene material from the tibial insert 30. If the distance between the two central fixation elements 1 is always, the same, tibial inserts 30 of different sizes can be used in assembling the artificial knee joint 100.

The orientation of the central fixation element 1 on the proximal surface P enables a linear guiding of the tibial insert 30 during the assembly (see Fig. 5), so that the central fixation elements 1 could also be termed as central guide rails. Furthermore, the central fixation elements 1 allow for an increased stability against shear forces in the artificial knee joint.

The proximal surface P also comprises an anterior fixation element 2 which is located at the anterior rim of the proximal surface P. The posterior side of the anterior fixation element 2 comprises a posterior wall 4 which is positioned perpendicular to the medial plane M of the proximal surface P. Therefore, the posterior wall 4 is also essentially perpendicular to the two linear central fixation elements 1.

The anterior fixation element 2 comprises two cavities 5, each in the form of a channel (i.e. the channel being open at both ends) with an essentially rectangular cross-section. As will be shown in connection with Fig. 5, those cavities are form fitting elements 5 cooperating with matching form fitting elements 31 on the tibial insert 30. The form fitting elements 5 in the anterior fixation element are positioned symmetrical relative to the medial plane M.

In other embodiments, the anterior fixation element 2 comprises only one or more than two form fitting elements 5. The cross-section of the cavities of the form fitting elements 5 does not have to be rectangular, as e.g. round or polygonal cross-sections can also be used.

In the embodiment of Fig. 2, the anterior fixation element 2 is one continuous element at the anterior rim of the tibial tray 20. In other embodiments, the anterior fixation element 2 can be divided into more than one part, e.g. two elements, each of them comprising a form fitting element 5.

The two form fitting elements 5 in the anterior fixation element 2 are positioned in a region in which the mechanical load is at less than 50% from the maximum load. This is due to the fact that the channel-like form fitting elements 5 somewhat weaken the structure of the anterior fixation element 1.

A further structure on the proximal surface P is a posterior fixation element 3, which is positioned at the posterior rim of the proximal surface P. The posterior fixation element 3 is positioned symmetrically relative to the medial plane M and comprises a posterior wall 7 flush with the posterior rim 8 of the tibial tray 20. The posterior wall 7 is following the curved shape of the posterior rim 8 of the tibial tray 20. The cross-section of the anterior fixation element 3 in a plane parallel to the proximal surface P is symmetric to the medial plane M.

The embodiment shown in Fig. 2 also comprises an anterior wall 9 which is parallel to the posterior wall 4 of the anterior fixation element 2. Therefore, the two central fixation elements 1 are oriented perpendicular to both the posterior wall 4 of the anterior fixation element 2 and the anterior wall 9 of the posterior fixation element 3.

The posterior fixation element 3 also comprises two sidewalls 10 which are angled relative to the anterior wall 9 of the posterior fixation element 3. The angle α between the two sidewalls 10 and the anterior wall 9 in a plane parallel to the proximal plane P is in the range between 5 and 85°, in particular in the range between 40 and 50°. Therefore, the two sidewalls 10 are oriented convergent in the anterior direction. The overall horizontal shape of the posterior fixation element 3 is roughly trapezoidal, with the long side of the trapezoid being curved.

The two sidewalls 10 of the posterior element 3 are tilted inwards by an angle between 1 and 25°, in particular between 3 and 10° relative to a plane which is perpendicular to the proximal plane P. Therefore, the top surface of the posterior fixation element 3 is slightly smaller than the base cross-section in the plane of the proximal surface.

In the embodiment described herein, and best shown in Fig. 5B, the anterior wall 9 of the posterior fixation element 3 comprises an undercut 6, the undercut 6 forming an angle β between 10 and 30°, in particular 15° with a plane perpendicular to the proximal surface P. In other embodiments, the undercut 6 can have - at least in parts - a curved shape. It is in principle also possible that the posterior wall 4 of the anterior fixation element 2 comprises an undercut 6.

In the embodiments discussed so far, the height H of the fixation elements 1, 2, 3 above the proximal surface P was identical. That does not have to be the case in all embodiments.

The matching tibial tray 20 and tibial insert 30 are shown side by side in Fig. 3A, 3B. The embodiment of the tibial tray 20 in Fig. 3A is already described in Fig. 2, so reference can be made to the respective description. The distal surface D of the tibia insert 30 is shown in Fig. 3B. The comparison of the two parts 20, 30 shows that the tibial insert 30 can be connected with the tibial tray 20 so that the first fixation elements 1 fit into matching grooves 34 in the tibial insert 30.

The tibial insert 30 comprises a recess 32 in the distal surface D for the matching posterior fixation element 3 of the tibial tray 20. The recess 32 is open towards the posterior side so that it only has an anterior wall 35 and two sidewalls 36.

The sidewalls 36 of the recess 32 are angled to match the angled sidewalls 10 of the posterior fixation element 3. In the corners where the anterior wall 35 and the two sidewalls 36 of the recess 32 meet, stress relief notches 33 are located at the junction of the two walls 35, 36.

At the anterior side of the tibial tray insert 30 two form fitting elements 31 are positioned which can be inserted into the two cavities of the form fitting elements 5 in the anterior fixation element 2 (see Fig. 2).

The grooves 34 and the recess 32 of the tibial tray 30 also form some form fitting connection with the matching structures, i.e. the central fixation elements 1 and the posterior fixation element 3 of the tibial tray 20. But the main function of the structures 32, 34 is an assistance during the assembly which will be described below in connection with Fig. 5A, 5B.

The connection between the tibial tray 20 and the tibial insert 30 is primarily effected by a light press fit connection between the tibial insert 30 and the at least two central fixation elements 1, the at least one anterior fixation element 2 and the at least one posterior fixation element 3, in particular having a press fit in the range of 0 to 250 µm. In Fig. 4, the surfaces 37 for the press fit at the posterior wall 4 of the anterior fixation element 2, at the inside of the central fixation elements 1 and the anterior wall 9 of the posterior fixation element 9 are highlighted. It is possible, that all press fit connections are dimensioned identically, but that does not have to be the case for all embodiments. For example the press fit between the posterior wall 4 of the anterior fixation element 2 and the anterior wall 9 of the posterior fixation element 3 is in the range of 25 to 125 µm and / or the press fit between the central fixation elements 1 is in the range of 0 and 100 µm.

In Fig. 5A and 5B the assembly of embodiments of a tibial insert 30 and a tibial tray is shown in different views.

Fig. 5A shows a perspective view from the front with the tibial insert 30 partially connected with the tibial tray 20 underneath. Fig. 5B shows essentially the same relative position of the tibial tray 20 and the tibial insert 30 but in a sectional view, with the anterior fixation element 2 on the right hand side.

The tibial insert 30 is first connected with the posterior fixation element 3 (see Fig. 2, 3A, 3B) which is used as some kind of fulcrum in the snapping into place. In the view of Fig. 5B the undercut 6 in the anterior wall 9 with an angle of about 15° is shown which allows a secure positioning of the tibial insert 30. At the anterior side (see Fig. 5A), the two form fitting elements 31 are shown which snap into the cavities of the form fitting element 5 (not seen in Fig. 5A).

In Fig. 6A, a first embodiment of a tibial insert 30 is shown in a frontal view. In this embodiment, the shape of the distal surface D is not fitted to the footprint, i.e. the outer rim of the tibial insert 30 is essentially a slightly inclined wall. The inclination can be between 0 and 10°. In case of an inclination of 0° the wall is straight.

In Fig. 6B, a second embodiment of a tibial insert 30 is also shown in a frontal view. Here, the shape of the distal surface D is fitted to the footprint, i.e. the outer wall is lightly tilted outwards.

Even though, the above described ceramic knee joint 100 with a metal-free, in particular ceramic, tibial tray 20 is a preferred embodiment, it is possible that the tibial tray 20 or the complete knee joint 100 are made from metal or from polymer material or that they comprise these materials.

### Reference signs

- 1: central fixation element
- 2: anterior fixation element
- 3: posterior fixation element
- 4: posterior wall of anterior fixation element
- 5: form fitting element of anterior fixation element
- 6: undercut in wall of posterior fixation element
- 7: posterior wall of posterior fixation element
- 8: posterior rim of tibial tray
- 9: anterior wall of posterior fixation element
- 10: sidewall of posterior fixation element

- 20: tibial tray

- 30: tibial insert
- 31: form fitting element for anterior fixation element
- 32: recess for posterior fixation element
- 33: relief notch
- 34: groove in tibial insert for linear central fixation element
- 35: anterior wall of recess
- 36: sidewall of recess
- 37: press fit surface

- 40: femur part

- 100: artificial knee joint

- D: distal surface of tibial insert
- H: height of a fixation element
- L1: lateral point of the proximal surface
- L2: lateral point of the proximal surface
- M: medial plane of proximal surface of tibial tray
- N: frontal plane perpendicular to the medial plane
- P: proximal surface of tibial tray
- α: angle between sidewalls and the anterior wall of the anterior fixation element in a plane parallel to the proximal surface
- β: angle of undercut

## Claims

1. Tibial tray (20) with a proximal surface (P), the proximal surface (P) comprising at least two, in particular exactly two central fixation elements (1) for enabling a connection with a tibial insert (30) as a further part of an artificial knee joint, wherein the at least two central fixation elements (1) are protrusions from the proximal surface (P) in the proximal direction and the at least two central fixation elements (1) are positioned symmetrically and / or parallel to a medial plane (M) of the proximal surface (P), the medial plane (M) being perpendicular to the proximal surface (P) and intersecting the proximal surface (P) in the middle between two most lateral points (L1, L2) of the proximal surface (P).

2. Tibial tray (20) according to claim 1, wherein at least one of the at least two central fixation elements (1) is cylindrical protrusion or a linear protrusion from the proximal surface (P).

3. Tibial tray (20) according to claim 1 or 2, wherein the at least two central fixation elements (1) are symmetrically positioned relative to a frontal plane (N) perpendicular to the medial plane (M), the frontal plane (N) in particular going through the most lateral points (L1, L2) of the proximal surface (P).

4. Tibial tray (20) according to any of the preceding claims, wherein at least one anterior fixation element (2), in particular exactly one anterior fixation element (2), is positioned at the anterior side of the proximal surface (P), the posterior side of the at least one anterior fixation element (2) comprising a wall (4) which in a plane parallel to the proximal surface (P) is perpendicular to the medial plane (M) of the proximal surface (P).

5. Tibial tray (20) according to claim 3, wherein the at least one anterior fixation element (2) comprises at least one form fitting element (5), in particular at least one cavity in the posterior wall (4) of the at least one anterior fixation element (2) and / or at least one channel through the at least one anterior fixation element (2), for a corresponding form fitting element (31) of the tibial insert (30), the at least one form fitting element (5) of the at least one anterior fixation element (2) being in particular positioned in a region in which the load is at less than 70%, in particular 50% from the maximum load.

6. Tibial tray (20) according to at least one of the preceding claims, wherein at least one, in particular exactly one posterior fixation element (3) is positioned symmetrically to the medial plane (M) of the proximal surface (P) comprising a posterior wall (7) flush with the posterior rim (8) of the tibial tray (20) and / or comprising an anterior wall (9) which is positioned parallel to the posterior wall (4) of the at least one anterior fixation element (2).

7. Tibial tray (20) according to claim 5, wherein the at least one posterior fixation element (3) comprises two sidewalls (10), an angle (a) between the sidewalls (10) and the anterior wall (9) in a plane parallel to the proximal plane (P) is in the range between 5 and 85°, in particular in the range between 40 and 50°.

8. Tibial tray (20) according to claim 5 or 6, wherein the sidewalls (10) of the at least one posterior element (3) are tilted by an angle between 1 and 25°, in particular between 3 and 10° relative to a plane which is perpendicular to the proximal plane (P).

9. Tibial tray (20) according to at least one of the claims 5 to 7, wherein the posterior wall (4) of the at least one anterior fixation element (2) and / or the anterior wall (9) of the at least one posterior fixation element (3) comprises an undercut (6), in particular the undercut (6) forming an angle (β) between 10 and 30°, in particular 15° with a plane perpendicular to the proximal surface (P).

10. Tibial tray (20) according to at least one of the preceding claims, wherein the height (H) of the at least two central fixation elements (1), the at least one anterior fixation element (2) and / or the at least one posterior fixation element (3) from the proximal surface (P) is in the range between 1 mm and 6 mm, in particular between 3 and 4,5 mm and / or wherein the anterior and / or posterior ends of the at least two central fixation elements (1) are rounded.

11. Tibial tray (20) according to at least one of the preceding claims, wherein the height (H) of the at least two central fixation elements (1), the height of the at least one anterior fixation element (2) and / or the height of the at least one posterior fixation element (3) over the proximal surface (P) is constant or varies by maximal 10% from the minimal height.

12. Tibial tray (20) according to at least one of the preceding claims, wherein it is manufactured completely or in part from ceramic, a polymer material or a metal.

13. Tibial insert (30) designed to match the tibial tray (20) of at least one of the preceding claims.

14. Tibial insert (30) according to claim 13, comprising a recess (32) in the distal surface (D) for matching at least one posterior fixation element (3), the recess (32) comprising at least one stress relief notch (33) at the junction of two walls (35, 36).

15. Tibial insert (30) according to claim 13 or 14, comprising at least one form fitting element (31) for engaging the at least one form fitting element (5) of the at least one anterior fixation element (2) of the tibial tray (20).

16. Artificial knee joint (100) comprising a tibial tray (10) according to at least one of the claims 1 to 13 and a tibial insert (30) according to at least one of the claims 11 to 13, wherein there is a light press fit connection between the tibial insert (30) and the at least two central fixation elements (1), the at least one anterior fixation element (2) and the at least one posterior fixation element (3) in in particular the press fit between the posterior wall (4) of the anterior fixation element (2) and the anterior wall (9) of the posterior fixation element (3) is in the range of 25 to 125 µm and / or the press fit between the central fixation elements (1) is in the range of 0 and 100 µm.
